# EUROPÄISCHE PATENTSCHRIFT

(11) **EP 0 707 006 B1**
(45) Veröffentlichungstag und Bekanntmachung des Hinweises auf die Patenterteilung: **07.04.1999**
(21) Anmeldenummer: 95810628.8
(22) Anmeldetag: 05.10.1995
(51) Int. Cl.: C07D 401/12, C07D 211/58, A61K 31/47, A61K 31/505, A61K 31/445

(54) **Aroyl-piperidin-Derivate**
Aroyl-piperidine derivatives
Dérivés d'aroyl-pipéridine

(30) Priorität: 14.10.1994 CH 3091/94
(43) Veröffentlichungstag der Anmeldung: 17.04.1996
(73) Patentinhaber: Novartis AG, 4058 Basel (CH); Novartis-Erfindungen Verwaltungsgesellschaft m.b.H., 1235 Wien (AT)
(72) Erfinder: Ofner, Silvio, Dr., CH-4142 Münchenstein (CH); Veenstra, Siem Jacob, Dr., CH-4053 Basel (CH); Schilling, Walter, Dr., CH-4204 Himmelried (CH)

(56) Entgegenhaltungen:
- EP-A- 0 532 456
- J.A.Lowe et al., DN & P, Mai 1992, Seiten 223-8

## Beschreibung

Die Erfindung betrifft neue N-(3,5-Bistrifluormethyl-benzoyl)-2-benzyl-4-(azanaphthoylamino)-piperidine der Formel 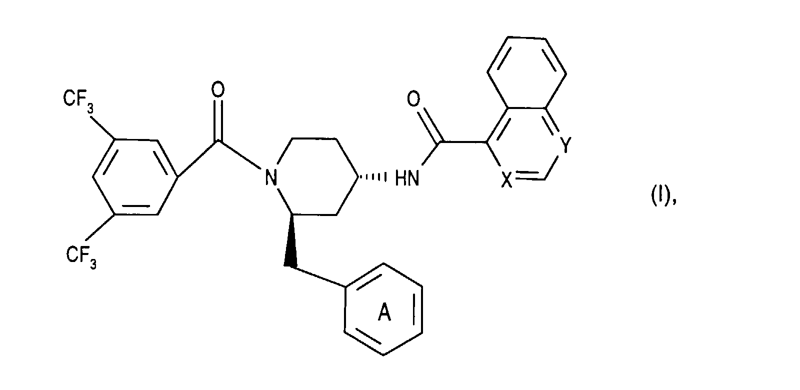 worin X und Y unabhängig voneinander für N oder CH stehen und der Ring A unsubstituiert oder ein- oder mehrfach substituiert ist durch Substituenten ausgewählt aus der Gruppe bestehend aus Niederalkyl, Niederalkoxy, Halogen, Nitro und Trifluormethyl; und ihre Salze, ihre Verwendung, Verfahren zur Herstellung und pharmazeutische Präparate enthaltend eine erfindungsgemässe Verbindung oder ein pharmazeutisch verwendbares Salz davon. (Die Bezeichnung "nieder" wird weiter unten definiert.)

Die Verbindungen der Formel I können als, insbesondere pharmazeutisch verwendbare, Salze vorliegen. Mit dem basischen Zentrum des Piperidinrings können Säureadditionssalze gebildet werden. Als Säurekomponente kommen beispielsweise starke anorganische Säuren, wie Mineralsäuren, z.B. Schwefelsäure, Phosphorsäuren, z.B. Orthophosphorsäure, Halogenwasserstoffsäuren, z.B. Chlorwasserstoffsäure, oder starke organische Carbonsäuren, wie gegebenenfalls, z.B. durch Halogen, substituierte Niederalkancarbonsäuren, z.B. Essigsäure oder Trifluoressigsäure, gegebenenfalls ungesättigte Dicarbonsäuren, z.B. Oxal-, Malon-, Bernstein-, Malein-, Fumar-, Phthal- oder oder Terephthalsäure, Hydroxycarbonsäuren, z.B. Ascorbin-, Glykol-, Milch-, Äpfel-, Wein- oder Zitronensäure, Aminosäuren, z.B. Asparagin- oder Glutaminsäure, oder Benzoesäure, oder organische Sulfonsäuren, wie gegebenenfalls, z.B durch Halogen, substituierte Niederalkansulfonsäuren, z.B. Methansulfonsäure, oder gegebenenfalls, z.B. durch Niederalkyl, substituierte Arylsulfonsäuren, z.B. p-Toluolsulfonsäure, in Betracht. Umfasst sind ferner für die therapeutische Verwendung nicht geeignete Salze, die beispielsweise für die Isolierung bzw. Reinigung von freien Verbindungen der Formel I oder deren pharmazeuisch verwendbaren Salzen eingesetzt werden können. Zur therapeutischen Anwendung gelangen nur die pharmazeutisch verwendbaren, nicht-toxischen Salze, die deshalb bevorzugt sind.

Da die erfindungsgemässen Verbindungen mindestens zwei optisch aktive Kohlenstoffatome aufweisen, können sie dementsprechend in Form von Stereoisomeren, Stereoisomerengemischen sowie in Form der (im wesentlichen) reinen Diastereomeren vorliegen. Entsprechende Stereoisomere sind ebenfalls von der vorliegenden Erfindung umfasst.

Bevorzugt sind solche Verbindungen der Formel I, worin der Ring A substituiert ist.

Die vor- und nachstehend verwendeten Allgemeinbegriffe haben, sofern nicht abweichend definiert, die nachfolgend angegebenen Bedeutungen.

Der Ausdruck "nieder" bedeutet, dass entsprechende Gruppen und Verbindungen jeweils 1 bis und mit 7, vorzugsweise 1 bis und mit 4, C-Atome enthalten.

Niederalkyl ist z.B. Methyl, Ethyl, n-Propyl, Isopropyl, n-Butyl, Isobutyl, sek-Butyl, tert-Butyl oder ein entsprechender Pentyl-, Hexyl- oder Heptylrest. Bevorzugt ist C₁-C₄-Alkyl.

Niederalkoxy ist z.B. Methoxy, Ethoxy, n-Propyloxy, lsopropyloxy, n-Butyloxy, lsobutyloxy, sec-Butyloxy, tert-Butyloxy oder ein entsprechender Pentyloxy-, Hexyloxy-, oder Heptyloxyrest. Bevorzugt ist C₁-C₄-Alkoxy.

Halogen ist insbesondere Halogen mit einer Atomnummer bis und mit 35, d.h. Fluor, Chlor oder Brom, und umfasst ferner lod. Bevorzugt ist Chlor.

Substanz P ist ein natürlich vorkommendes Undekapeptid der Tachykininfamilie. Es wird in Säugetierorganismus erzeugt und wirkt pharmakologisch als Neuropeptid. Substanz P spielt eine wesentliche Rolle bei verschiedenen Erkrankungen, beispielsweise bei Schmerzzuständen, bei Migräne und bei einigen Störungen des Zentralnervensystems, wie bei Angstzuständen, Emesis, Schizophrenie und Depressionen sowie bei bestimmten motorischen Störungen, wie bei morbus Parkinson, aber auch bei entzündlichen Erkrankungen, wie bei rheumatoider Arthritis, Iritis und Konjunktivitis, bei Erkrankungen der Atmungsorgane, wie bei Asthma und chronischer Bronchitis, bei Erkrankungen des Gastrointestinalsystems, wie bei ulcerativer Colitis und morbus Crohn, und bei Hypertension.

Das Ziel vielfacher Bemühungen ist es, die Entwicklung auf dem Gebiet der Substanz-P-Antagonisten weiterzutreiben und beispielseise geeignete Substanz-P-Antagonisten mit einem breiten Wirkungsspektrum zu finden, die sowohl eine ausgezeichnete in vivo Aktivität und eine erhöhte Bioverfügbarkeit, als auch eine verbesserte chemische Stabilität aufweisen.

Ausgedehnte pharmakologische Untersuchungen haben ergeben, dass die erfindungsgemässen Verbindungen und ihre Salze in besonders bevorzugtem Masse Substanz P antagonisieren und somit die durch Substanz P ausgelösten Krankheitserscheinungen verhindern.

Die Substanz-P-antagonisierenden Effekte können - wie nachstehend dargelegt - unter Heranziehung von dem Fachmann bekannten Testmethoden nachgewiesen werden. Derartige Effekte werden sowohl *in vitro* als auch *in vivo* gefunden. So wird durch die Verbindungen der Formel I bzw. lA und ihre pharmazeutisch verwendbaren Salze *in vitro* die durch Substanz P induzierte Bildung von Phosphoinositol in menschlichen Astrocytoma Zellen antagonisiert. Dabei ergeben sich IC₅₀-Werte ab etwa 1 nM. Als Testmodell für den Nachweis dieser Hemmung eignet sich beispielsweise die Testmethode von Lee, C.M. et al., wie sie in *J.Neurochem.* 59, 406-414 (1992) beschrieben wird.

Weiterhin wird die Bindung von ³H-Substanz- P an der Rinder-Retina im Radiorezeptor-Assay nach H. Bittiger, Ciba Foundation Symposium 91, 196-199 (1982) mit IC₅₀-Werten ab etwa 1 nM gehemmt. . So wurden beispielsweise für die Zielverbindungen der Beispiele 3, 4 und 5 folgende in-vitro-Werte ermittelt: 7nM - 6nM - 6.9nM.

Durch i.c.v.-Verabreichung von Substanz-P-methylester bei Wüstenspringmäusen (gerbil) wird eine Verhaltensänderung hervorgerufen. Dieser Effekt kann nach peroraler Verabreichung von Verbindungen der Formel I bzw. lA und ihren Salzen *in vivo* gehemmt werden. Als Testmethodik wird das Verfahren von A.Vassout et al. verwendet, welches am Kongress "Substanz P and Related Peptides: Cellular and Molecular Physiology" in Worchester, Mass., 1990 vorgestellt wurde. Dabei werden ED₅₀-Werte ab etwa 0.1 mg/kg p.o. ermittelt. Damit ergibt sich ihre Verwendbarkeit für die Behandlung von Erkrankungen des zentralen Nervensystems.

*In vivo* hemmen die Verbindungen der Formel I bzw. lA und ihre Salze ab einer Dosis von etwa 1.0 mg/kg i.v., in Anlehnung an die Versuchsanordnung von Lundberg et al, Proc. Nat. Acad. Sci. (USA) 80, 1120-1124, vagal induzierte Bronchospasmen des Meerschweinchens, woraus sich ihre Eignung für die Behandlung von Asthma ergibt.

Gegenüber den entsprechenden in der Europäischen Patentanmeldung mit der Veröffentlichungs-Nr. 532 456 A1 offenbarten Verbindungen weisen die erfindungsgemässen Verbindungen nicht nur eine deutlich bessere in vivo Aktivität auf, sondern sie zeigen auch eine wesentlich höhere chemische Stabilität, z.B. gegenüber Sauerstoff, sowie eine gesteigerte orale Bioverfügbarkeit.

Die erfindungsgemäss bereitgestellten Substanz-P-Antagonisten der Formel I bzw. lA und ihre pharmazeutisch verwendbaren Salze eignen sich dementsprechend vorzüglich zur therapeutischen Behandlung der vorstehend aufgeführten pathologischen Erscheinungen.

Die Erfindung betrifft auch die Verwendung einer Verbindung der Formel I bzw. lA oder eines pharmazeutisch verwendbaren Salzes davon zur Herstellung von Arzneimitteln zur Behandlung von Erkrankungen, die durch Substanz P ausgelöst werden.

Die Erfindung betrifft weiterhin die Verwendung von Verbindungen der Formel I bzw. lA oder eines Salzes davon als biochemische Hilfsmittel (tools), beispielsweise für die Identifizierung und ggf. zur Profilierung von weiteren potenten Substanz-P-Antagonisten.

Die Erfindung betrifft in erster Linie Verbindungen der Formel lA 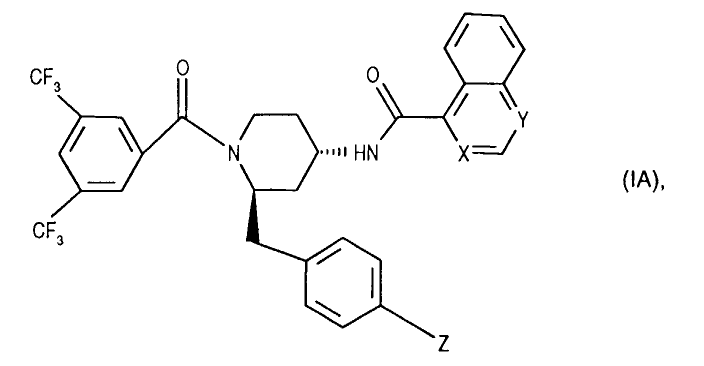 worin X für CH oder N steht und Y N ist; und Z Wasserstoff, Halogen oder Nitro bedeutet; und ihre Salze.

Die Erfindung betrifft in allererster Linie Verbindungen der Formel lA, worin X für N oder CH steht und Y N ist; und Z Halogen, wie Chlor, bedeutet; und ihre Salze.

Die Erfindung betrifft namentlich die in den Beispielen genannten Verbindungen der Formel I und ihre Salze.

Die Erfindung betrifft ferner Verfahren zur Herstellung der erfindungsgemässen Verbindungen. Diese sind dadurch gekennzeichnet, dass man
a) eine Verbindung der Formel (lla) 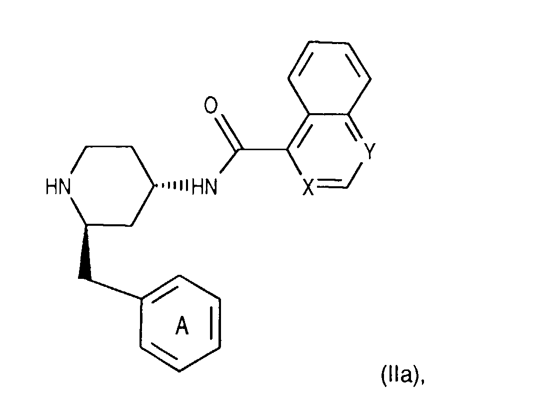 worin X und Y die angegebenen Bedeutungen haben und der Ring A unsubstituiert oder wie angegeben substituiert ist, mit einer Verbindung der Formel llb 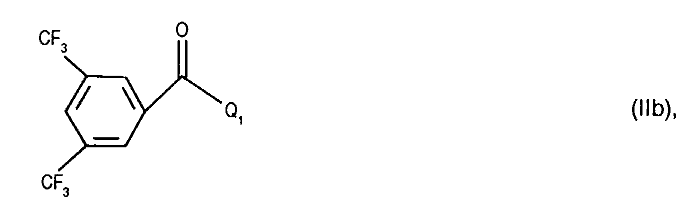 worin Q₁ gegebenenfalls verethertes Hydroxy, wie Hydroxy, Niederalkoxy oder gegebenenfalls substituiertes Phenoxy, oder reaktionsfähiges verestertes Hydroxy, wie Halogen, insbesondere Chlor, oder einen Rest der Formel 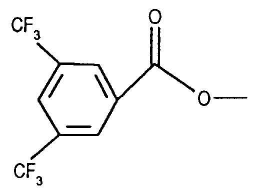 bedeutet, oder einem Salz davon umsetzt; oder
b) eine Verbindung der Formel (IIIa) 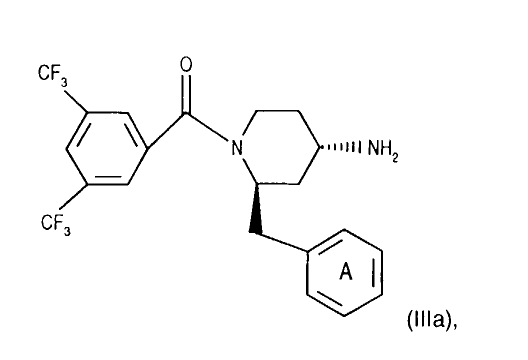 worin der Ring A unsubstituiert oder wie angegeben substituiert ist, mit einer Verbindung der Formel Illb 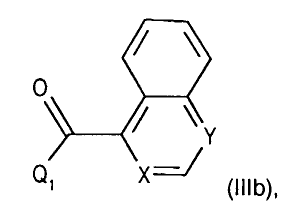 worin X und Y die angegebenen Bedeutungen haben und Q₁ gegebenenfalls verethertes Hydroxy, wie Hydroxy, Niederalkoxy oder gegebenenfalls substituiertes Phenoxy, oder reaktionsfähiges verestertes Hydroxy, wie Halogen, insbesondere Chlor, oder einen Rest der Formel 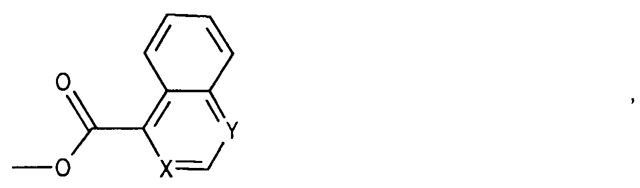 bedeutet, oder einem Salz davon umsetzt;
und, wenn erwünscht, ein verfahrensgemäss erhältliches Gemisch von Isomeren auftrennt und das gewünschte Isomere isoliert und/oder eine verfahrensgemäss erhältliche freie Verbindung I bzw. lA in ein Salz oder ein verfahrensgemäss erhältliches Salz einer Verbindung I bzw. lA in die freie Verbindung I bzw. lA oder in ein anderes Salz überführt.

Salze von Ausgangsmaterialien, die mindestens ein basisches Zentrum aufweisen, beispielsweise der Formel IIa oder IIIa, sind entsprechende Säureadditionssalze, während Salze von Ausgangsstoffen, die eine acide Gruppe aufweisen, beispielsweise der Formel IIb oder IIIb, als Salze mit Basen vorliegen, jeweils wie in Zusammenhang mit entsprechenden Salzen der Formel I bzw. IA vorstehend aufgeführt.

Die vor- und nachstehend in den Varianten beschriebenen Umsetzungen werden in an sich bekannter Weise durchgeführt, z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet.

### Verfahrensvariante a) bzw. b):

Die Kondensation zur Herstellung der jeweiligen Amidbindung kann in an sich bekannter Weise durchgeführt werden, beispielsweise wie in Standardwerken, wie "Houben-Weyl, Methoden der organischen Chemie", 4. Auflage, Band 15/11, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, femer geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoram idochloridat, Bis-(2-oxo-3-oxazolidinyl)-phosphinsäu rechlorid oder 1-Benzotriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation eines Carbonsäurehalogenids beispielsweise mit einem entsprechenden Amin kann auch in Gegenwart einer geeigneten Base ohne Zusatz einer geeigneten Kupplungskomponente erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Ether, z.B. Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden.

Das Ausgangsmaterial der Formel (IIb) und (IIIb) ist bekannt oder kann in an sich bekannter Weise hergestellt werden.

Verbindungen der Formel (IIIa) können in an sich bekannter Weise hergestellt werden. Beispielsweise geht man von einer Verbindung der Formel 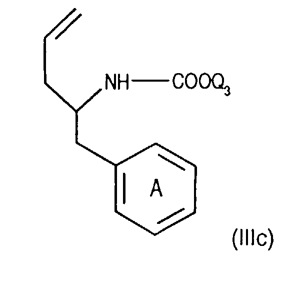 aus, worin Q₃ beispielsweise Niederalkyl oder Phenylniederalkyl bedeutet, N-alkyliert z.B. durch Umsetzung mit Niederalkoxy-halogen-methan, wie Ethoxy-chlormethan, in Gegenwa einer Base und behandelt die so erhältliche Verbindung der Formel (IIId) 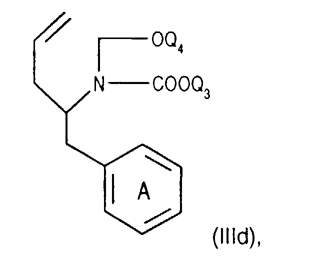 worin Q₄ z.B. Niederalkyl bedeutet, mit einem Nitril, wie Acetonitril, in Gegenwart einer starken Säure, wie Chlorsulfonsäure. In der so resultierenden Verbindung der Formel (Ille) 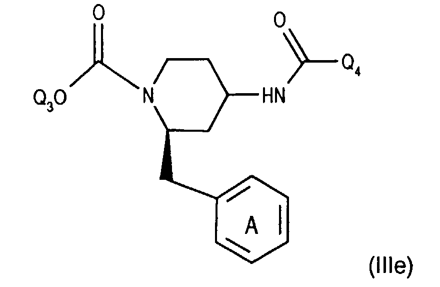 wird die -C(=O)-OQ₃-Gruppe durch Behandeln mit einer starken Säure, z.B. mit Bromwasserstoffsäure, abgespalten.
Zur Herstellung einer enantiomerenreinen Verbindung wird in einer so erhältlichen Verbindung der Formel (IIIf) 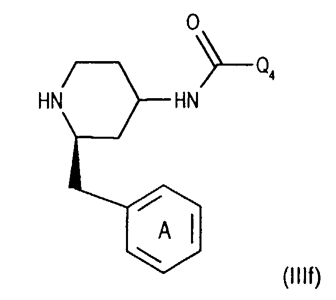 die sekundäre Aminogruppe mit einer optisch aktiven Verbindung, wie einer entsprechenden O-acylierten α-Hydroxycarbonsäure oder einem reaktionsfähigen Derivat davon, z.B. O-Acetyl-(+)mandelsäure-chlorid, acyliert und das so zugängliche Diastereomerengemisch in an sich bekannter Weise, z.B. chromatographisch, aufgespalten. Nach Abspaltung beider N-Acylgruppen, beispielsweise durch saure Hydrolyse, z.B. mit Salzsäure, erhält man eine Verbindung der Formel (IIIg)

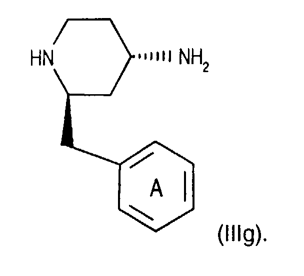

Die 4-Aminogruppe von Verbindungen der Formel (IIIg) wird vorübergehend in an sich bekannter Weise geschützt, beispielsweise durch Umsetzung mit Benzaldehyd. Anschliessend führt man die 3,5,-Bistrifluormethylbenzoyl-Gruppe ein, beispielsweise wie für Verfahrensvariante a) beschrieben durch Kupplung mit einer Verbindung der Formel (llb), und spaltet die Schutzgruppe der 4-Aminogruppe ab, beispielsweise durch Behandeln mit einer Säure, wie Salzsäure, und gelangt so zu einer entsprechenden Verbindung der Formel (IIIa).

Verbindungen der Formel (lla) können in an sich bekannter Weise hergestellt werden. Beispielsweise geht man von einer Verbindung der Formel (IIIg) aus und kuppelt diese, beispielsweise wie für Verfahrensvariante b) beschrieben, mit einer Verbindung der Formel (IIIb) in Gegenwart eines Kupplungsreagenzes und führt so die entsprechende (Aza)Naphthoyl-Gruppe ein. Man erhält so eine entsprechende Verbindung der Formel (IIa).

Die Erfindung wird insbesondere durch die Beispiele illustriert und betrifft auch die in den Beispielen genannten neuen Verbindungen sowie die Verfahren zur Herstellung.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I bzw. IA, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I bzw. IA enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I bzw. lA führende Ausgangsstoffauswahl, die Verfahren zu ihrer Herstellung und ihre Verwendung als Zwischenprodukte bilden ebenfalls einen Gegenstand der Erfindung, einschliesslich der Verbindung (2R*,4S*)-N-[1-(3,5-Bistrifluormethyl-benzoyl)-2-(4-nitro-benzyl)-piperidin-4-yl]-acetamid.

Die neuen Verbindungen der Formel I bzw. lA können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I bzw. lA in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I bzw. IA , vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Celsiusgraden, Drucke in mbar angegeben.

### Beispiel 1: (2R,4S)-N-[1-(3,5-Bistrifluormethyl-benzoyl)-2-benzyl-piperidin-4-yl]- chinolin-4-carboxamid

Zur Lösung von 4.16 g (9.67 mMol) (2R,4S)-2-Benzyl-1-(3,5-bis-trifluormethyl-benzoyl)-4-piperidinamin [*EP 532 456 A1, Beispiel 38 f)*], 1.84 g (10.6 mMol) Chinolin-4-carbonsäure und 3 ml (21.3 mMol) Triethylamin in 30 ml Methylenchlorid gibt man bei 0°C 2.95 g (11.6 mMol) Bis-(2-oxo-3-oxazolidinyl)-phosphinsäurechlorid und entfernt das Eisbad nach 10 Minuten. Man lässt das Reaktionsgemisch 4 Stunden bei Raumtemperatur rühren. Danach wird mit Methylenchlorid verdünnt, die organische Phase mit wässriger 10-prozentiger Zitronensäure und mit 1 N Kaliumcarbonatlösung extrahiert, mit Sole gewaschen, über Magnesiumsulfat getrocknet und zur Trockene eingedampft. Das Rohprodukt wird mit Methylenchlorid/Aceton (7:3) an Kieselgel chromatographiert. Aus Methylenchlorid/Ether/Hexan /Hexan kristallisiert die Titelverbindung als weisse Kristalle. Smp.: 187°C; DC: Methylenchlorid/Aceton (7:3) R_{f}= 0.48, FD-MS: M⁺= 585; [alpha]D = + 9.9 (c=1, Methylenchlorid); Analyse: ber.: C = 63.59%, H= 4.30%, N = 7.18%, F = 19.47%, gef.: C = 63.50%, H= 4.40%, N = 7.16%, F = 19.45%.

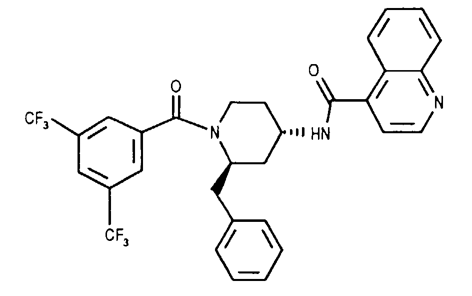

### Beispiel 2: (2R,4S)-N-[1-(3,5-Bistrifluormethyl-benzoyl)-2-benzyl-pipefldin-4-yl]-chinazolin-4-carboxamid

Analog Beispiel 1 werden 252 mg (1.45 mMol) (2R,4S)-2-Benzyl-1-(3,5-bis-trifluormethylbenzoyl)-4-piperidinamin in 5 ml Methylenchlorid mit 368 mg 1 (1.45 mMol) Bis-(2-oxo-3-oxazolidinyl)-phosphinsäurechlorid, 440 mg (4.35 mMol) Triethylamin und 566 mg (1.32 mMol) Chinazolin-4-carbonsäure umgesetzt. Man erhält die Titelverbindung als weisse Kristalle. Smp.: 92-172°C; DC: Hexan/Ethylacetat (1:1) R_{f}= 0.37, FD-MS: M⁺= 586; [alpha]D = + 1.3 (c=0.98, Methylenchlorid).

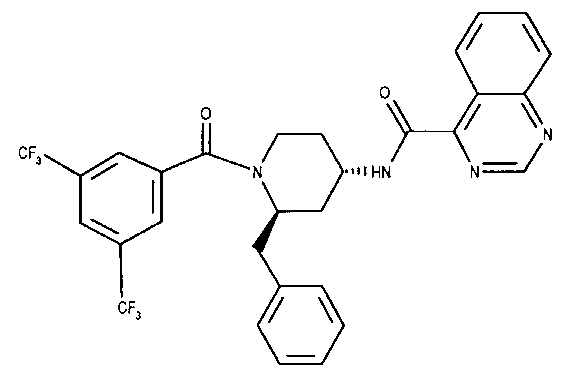

### Beispiel 3: (2R,4S)-N-[1-(3,5-Bistrifluormethyl-benzoyl)-2-(4-chlor-benzyl)-piperidin-4-yl]-chinolin-4-carboxamid

Zur einer Lösung von 0.915 g (4.01 mMol) Chinolin-4-carbonsäurechlorid-hydrochlorid [*Nicolaï E, Güngör T, Goyard J, Cure G, Fouquet A, Teulon JM, Delchambre C, Cloarec A, Eur. J. Med. Chem., 1992, 27, 977*] in 10 ml Methylenchlorid wird bei 0°C eine Lösung von 1.70 g (3.65 mMol) (2R,4S)-1-(3,5-Bis-trifluormethyl-benzoyl)-2-(4-chlor-benzyl)-piperidin-4-amin und 1.5 ml (10.96 mMol) Triethylamin in 3 ml Methylenchlorid getropft. Nach 15 Stunden werden nochmals 228 mg (1.0 mMol) Chinolin-4-carbonsäurechlorid-hydrochlorid und 0.1 ml (1.0 mMol) Triethylamin zugegeben und 3 Stunden gerührt. Das Gemisch wird mit 1N Salzsäure versetzt und mit Ethylacetat extrahiert. Die organische Phase wird mit 1 N Natronlauge und mit Sole gewaschen, über Natriumsulfat getrocknet und eingeengt. Der Rückstand wird aus Toluol kristallisiert und ergibt die Titelverbindung als weisse Kristalle. Smp. 204-207°C (Phasenübergang 135-140°C); DC: Ethylacetat: R_{f}= 0.48, FD-MS: M⁺= 619(621).

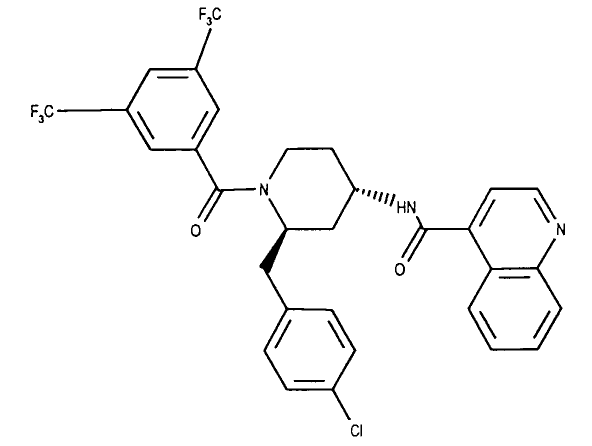

Das Ausgangsmaterial kann beispielsweise wie folgt hergestellt werden:
a) N-[1-(4-Chlor-benzyl)-but-3-enyl]-N-ethoxymethyl-carbaminsäuremethylester
   Eine Suspension von 10.0 g Natriumhydrid (80% in Mineralöl, 333 mMol) in trockenem Tetrahydrofuran [THF] unter Argon wird auf Rückfluss erhitzt. Eine Lösung von 60.5 g (238 mMol) [1-(4-Chlor-benzyl)-but-3-enyl]-carbaminsäuremethylester [*McCarty FJ, Rosenstock PD, Paolini JP, Micucci DD, Ashton L, Bennetts WW, J. Med. Chem, 1968, 11(3),534*] in 50 ml trockenem THF wird innerhalb 1 Stunde zugetropft. Anschliessend wird das Gemisch noch 2 Stunden unter Rückfluss gekocht bis die Gasentwicklung nachlässt. Das Gemisch wird auf 0°C abgekühlt und Chlormethylethylether zugetropft, so dass die Reaktionstemperatur nicht über 5°C steigt. Nachher wird langsam auf 25°C erwärmt und 12 Stunden ausgerührt. Überschüssiges Natriumhydrid wird vorsichtig mit 1 ml Wasser vernichtet bevor man mehr Wasser zugibt. Die Phasen werden getrennt und die Wasserphase nochmals mit tert-Butylmethylether extrahiert. Die kombinierten organischen Phasen werden mit Sole gewaschen, über Natriumsulfat getrocknet und eingedampft. Das Rohprodukt wird bei 0.1 mbar destilliert und hat einen Siedebereich von 120-125°C. DC: Ethylacetat/Hexan (1 : 6) R_{f}= 0.34, FD-MS: M⁺= 311(313).
b) (2R*,4S*)-4-Acetylamino-2-(4-chlor-benzyl)-piperidin-1-carbonsäure-methylester
   Bei -40°C werden 20.6 ml (308 mMol) Chlorsulfonsäure in 500 ml Acetonitril vorgelegt. Eine Lösung von 48.0 g (154 mMol) N-[1-(4-Chlor-benzyl)-but-3-enyl]-N-ethoxymethyl-carbaminsäuremethylester in 50 ml Acetonitril wird zugetropft, so dass die Reaktionstemperatur nicht über -10°C hinaussteigt. Anschliessend wird noch 20 Minuten bei -15°C gerührt, bevor man die Reaktion mit 370 ml 2N Natronlauge und 100 ml 10%-ige wässrige Natriumhydrogencarbonatlösung versetzt. Die Phasen werden getrennt und die wässrige Phase noch zweimal mit Toluol extrahiert. Die kombinierten organischen Phasen werden über Natriumsulfat getrocknet. Das Rohprodukt wird aus Toluol kristallisiert und ergibt die Titelverbindung als weisse Kristalle. Smp.: 169-170°C. DC: Methylenchlorid/Methanol/konz. Ammoniak (90 : 9 :1) R_{f}= 0.42, FD-MS: M⁺= 325.
c) (2R*,4S*) N-[2-(4-Chlor-benzyl)-piperidin-4-yl]-acetamid
   (2R*,4S*)-4-Acetylamino-2-(4-chlor-benzyl)-piperidin-1-carbonsäure-methylester (30.0 g, 92.3 mMol) wird mit 51.8 ml 33 prozentigem Bromwasserstoff in Essigsäure versetzt. Nach 16 Stunden wird das Gemisch mit 200 ml Wasser versetzt und zweimal mit Toluol gewaschen. Die Wasserphase wird basisch gestellt und zweimal mit Essigsäureethylester extrahiert. Die organischen Phasen werden auf Kaliumcarbonat getrocknet und am Rotationsverdampfer eingedampft. Die Titelverbindung kristallisiert als Hydrochlorid aus EtOH/Ethylacetat. Smp.: 288-289°C. DC: Methylenchlorid/Methanol/konz. Ammoniak (90:9:1) R_{f}= 0.17, FD-MS: (M+1)⁺= 267.
d) (2'S,2R,4S)-Essigsäure-2-[4-acetylamino-2-(4-chlor-benzyl)-piperidin-1-yl]-2-oxo-1-phenyl-ethylester
   Racemisches N-[2-(4-Chlor-benzyl)-piperidin-4-yl]-acetamid-hydrochlorid (20.5 g, 67.6 mMol) wird unter kräftigem Rühren bei 0°C in 34 ml 2N Natronlauge, 150 ml einer 10 prozentigen wässrigen Natriumhydrogencarbonatlösung und 50 ml Methylenchlorid vorgelegt. Man tropft während 1 Stunde. S(+)-O-Acetyl-mandelsäurechlorid [*Pracejus G, Ann., 1959, 622, 10*] (14.9 g, 70 mMol) dazu. Anschliessend wird 1 Stunde bei +4°C nachgerührt. Die Phasen werden getrennt, die organische Phase über Natriumsulfat getrocknet und am Rotationsverdampfer eingedampft. Die Titelverbindung wird als reines Diastereomer nach zweifachem Kristallisieren aus Methylenchlorid /tert-Butylmethylether isoliert. Smp.: 209-211°C. DC: Methylenchlorid/Isopropanol (9:1) R_{f}= 0.65, FD-MS: M⁺= 443. [alpha]D = + 77.5 Grad (c=1, Methylenchlorid).
   Die Mutterlaugen enthalten hauptsächlich das nicht-kristalline Diastereomer (2'S,2S,4R)N-[2-(4-Chlor-benzyl)-1-(acetoxy-phenyl-acetyl)pipeddin-4-yl]-acetamid. DC: Methylenchlorid/Isopropanol (9:1) R_{f}= 0.70.
e) (2R,4S)1-(3,5-Bistrifluormethyl-benzoyl)-2-(4-chlor-benzyl)-piperidin-4-amin
   (2'5,2R,4S)-Essigsäure-2-[4-acetylamino-2-(4-chlor-benzyl)-piperidin-1-yl]-2-oxo-1 -phenylethyl ester (37.4 g, 84.5 mMol) wird während 2 Tagen in 370 ml 6 N Salzsäure unter Rückfluss gekocht. Nach Abkühlen wird das Gemisch mit festem Natriumhydroxid basisch gestellt und mit Methylenchlorid extrahiert. Die kombinierten organischen Phasen werden über Kaliumcarbonat getrocknet und am Rotationsverdampfer eingedampft. Der Rückstand, bestehend aus fast reinem (2R,4S)-2-(4-Chlor-benzyl)-piperidin-4-amin (19.0 g, 84.5 mMol, 100%), wird mit 8.5 ml (84.5 mMol) Benzaldehyd versetzt und zwei Mal mit 150 ml Toluol am Rotationsverdampfer eingeengt. Der ölige Rückstand wird in 180 ml Methylenchlorid und 15.3 ml (110 mMol) Triethylamin aufgenommen und auf 10°C gekühlt.
   Bistrifluormethylbenzoylchlorid (25.7 g, 92.9 mMol) wird während 15 Minuten zugetropft und anschliessend 1 Stunde bei 25°C ausgerührt. Das Reaktionsgemisch wird mit 250 ml 1 N Salzsäure versetzt und das Methylenchlorid unter vermindertem Druck am Rotationsverdampfer entfernt. Hexan und Ethanol werden zugegeben bis zwei homogene Phasen entstehen. Nach Abtrennen der organischen Phase wird weiter mit Hexan gewaschen bis alles Benzaldehyd entfernt ist. Das Gemisch wird mit festem Natriumhydroxid basisch gestellt und wiederholt mit Methylenchlond extrahiert. Die organischen Phasen werden über Natriumsulfat getrocknet und am Rotationsverdampfer eingeengt. Kristallisation aus tert-Butylmethylether/Hexan /Hexan ergibt die Titelverbindung als weisse Kristalle. Smp.: 79-81°C. DC: Methylenchlorid/Methanol/konz. Ammoniak (90:9:1) R_{f}= 0.21, FD-MS: (M+1)⁺= 465. [alpha]D = - 12.7 Grad (c=1, Methylenchlorid).

### Beispiel 4: (2R,4S)-N-[1-(3,5-Bistrifluormethyl-benzoyl)-2-(4-chlor-benzyl)-piperidinyl]-chinazolin-4-carboxamid

Chinazolin-4-carbonsäure-ammoniumsalz (0.238 g, 1.25 mMol, [5]) wird in Methanol gelöst und mit 1 ml Triethylamin versetzt. Die Lösung wird am Rotationsverdampfer eingeengt. Dieser Vorgang wird zweimal wiederholt und ergibt Chinazolin-4-carbonsäuretriethylammoniumsalz als Öl, das zusammen mit 0.465 g (1.0 mMol) (2R,4s)1-(3,5-Bistrifluormethyl-benzoyl)-2-(4-chlor-benzyl)-piperidin-4-amin und 0.175 ml Triethylamin in 10 ml Methylenchlorid gelöst wird. Bei 0°C gibt man jetzt 0.316 g (1.25 mMol) Bis-(2-oxo-3-oxazolidinyl)-phosphinsäurechlorid dazu und entfernt nach 10 Minuten das Eisbad. Man lässt das Reaktionsgemisch 4 Stunden bei Raumtemperatur rühren. Danach wird mit Ethylacetat verdünnt, die organische Phase mit wässriger 10-prozentiger Natriumbicarbonatlösung und Sole gewaschen, über Natriumsulfat getrocknet und zur Trockene eingedampft. Das Rohprodukt wird mit Ethylacetat/Hexan (1:2) chromatographiert. Aus Ethylacetat/Hexan kristallisiert die Titelverbindung als weisse Kristalle. Smp.: 118-120°C; DC: Ethylacetat/Hexan (1 : 1) R_{f} = 0.42; FD-MS: M⁺= 620; [alpha]D = -15.7 (c=1, Methylenchlond); Analyse: ber.: C = 58.03%, H = 3.73%, N = 9.05%, gef.: C = 57.9%, H = 3.7%, N = 8.9%.

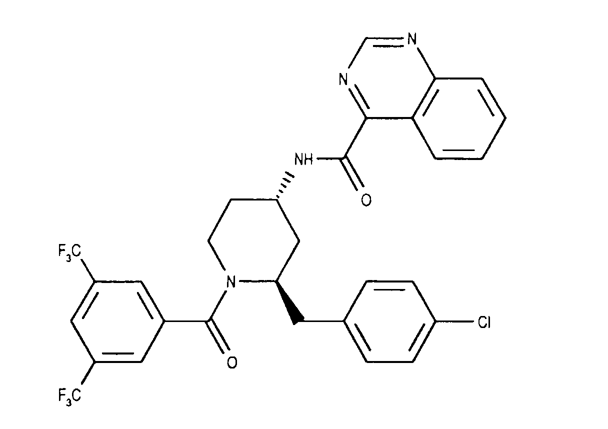

### Beispiel 5: (2R,4S)-N-[1-(3,5-Bistrifluormethyl-benzoyl)-2-(4-chlor-benzyl)-piperidinyl]-isochinolin-1-carboxamid

Analog Beispiel 1 werden 0.30 g (0.64 mMol) (2R,4S)1-(3,5-Bistrifluormethyl-benzoyl)-2-(4-chlor-benzyl)-piperidin-4-amin in 8 ml Methylenchlorid mit 0.18 g (0.71 mMol) Bis-(2-oxo-3-oxazolidinyl)-phosphinsäurechlorid, 0.18 ml (1.30 mMol) Triethylamin und 0.112 g (0.64 mMol) Isochinolin-1-carbonsäure umgesetzt. Das Rohprodukt wird mit Ethylacetat/Hexan (1:3) chromatographiert. Aus Ethylacetat/Hexan kristallisiert die Titelverbindung als weisse Kristalle. Smp.: 112-113°C; DC: Ethylacetat/Hexan (1:2) R_{f} = 0.30; FD-MS: M⁺= 619(621); [alpha]D = -11.3 (c=1, EtOH); Analyse: ber.: C = 60.06%, H = 3.90%, N = 6.78%, gef.: C = 61.0%, H = 4.5%, N = 6.4%.

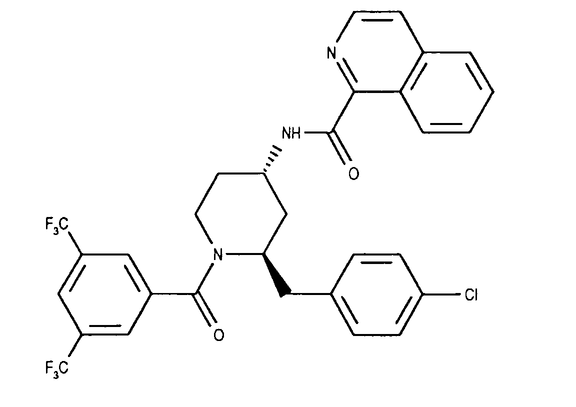

### Beispiel 6: (2R,4S)-N-[1-(3,5-Bistrifluormethyl-benzoyl)-2-(4-nitro-benzyl)-piperidinyl]-chinazolin-4-carboxamid

Analog Beispiel 4 werden 0.14 g (0.29 mMol) (2R*,4S*)1-(3,5-Bistrifluormethyl-benzoyl)-2-(4-nitro-benzyl)-piperidin-4-amin in 5 ml Methylenchlorid mit 0.083 g (0.32 mMol) Bis-(2-oxo-3-oxazolidinyl)-phosphinsäurechlorid, 0.11 ml (0.75 mMol) Triethylamin und 0.058 g (0.30 mMol) Chinazolin-4-carbonsäu re-ammoniumsalz [*Armarego WLF und Smith JIC*, *J. Chem. Soc. (B), 1967, 449*] umgesetzt. Das Rohprodukt wird mit Ethylacetat/Hexan (1:3) chromatographiert. Aus Ethylacetat/Hexan kristallisiert die Titelverbindung als weisse Kristalle. Smp.: 112-113°C; DC: Methylenchlorid/Methanol (19:1) R_{f} = 0.55; FD-MS: M⁺= 631.

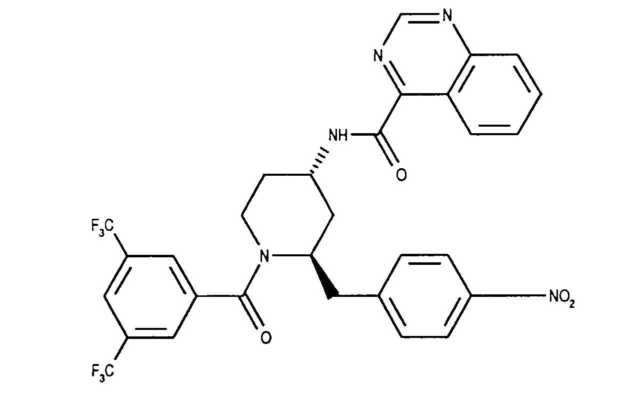

Das Ausgangsmaterial kann beispielsweise wie folgt hergestellt werden:
a) [1-(4-Nitro-benzyl)-but-3-enyl]-carbaminsäurebenzylester
   2-(4-Nitro-benzyl)-pent-4-encarbonsäure re [2-(4-Nitro-benzyl)-pent-4-ensäure wird analog hergestellt wie 2-(4-Chlor-benzyl)-pent-4-ensäure, vergl. *J. Med. Chem, 1968, 11(3),534*] (9.3 g, 40.0 mMol) wird in Anwesenheit von Triethylamin (5.6 ml, 40.0 mMol) und Benzylalkohol (5.18 g, 48.0 mMol) in Toluol mit 11.55 g (42.0 mMol) Phosphorsäure-diphenylesterazid bei 50°C versetzt. Nach 20 Minuten wird die Temperatur langsam erhöht und anschliessend wird noch 3 Stunden unter Rückfluss gehalten. Nach Abkühlen wird das Reaktionsgemisch zweimal mit 1 N Natronlauge, zweimal mit 1 N Salzsäure und zweimal mit Sole gewaschen, über Natriumsulfat getrocknet und am Rotationsverdampfer eingedampft. Die Titelverbindung wird als nach Kristallisation des Rohproduktes aus Toluol/Hexan weisse Kristalle erhalten. Smp: 94-95°C. DC: Ethylacetat/ Hexan (1:3) R_{f}= 0.22.
b) N-[1-(4-Nitro-benzyl)-but-3-enyl]-N-ethoxymethyl-carbaminsäurebenzylester
   Ein Gemisch aus 10 ml wässriger 50-prozentiger Natronlauge, 25 ml Methylenchlorid und 130 mg (0.4 mMol) Benzyltributylammoniumchlorid wird bei 5-10°C kräftig gerührt. Nach der Zugabe von [1-(4-Nitro-benzyl)-but-3-enyl]-carbaminsäure-benzylester (6.8 g, 20.0 mMol) werden 2.64 g (28 mMol) Chlormethylethylether während 2 Stunden zugetropft. Das Gemisch wird anschliessend 1 Stunde bei Raumtemperatur gerührt, mit Eis und Wasser verdünnt und mit Methylenchlorid extrahiert. Der organische Phase wird über Natriumsulfat getrocknet, zur Trockene eingedampft und an Kieselgel mit Hexan/Ethylacetat (10:1) chromatographiert. Die Titelverbindung wird als farbloses Öl erhalten. DC:
   Ethylacetat/Hexan (1:3) R_{f}= 0.41. FD-MS: M⁺: 398.
c) (2R*,4S*)-4-Acetylamino-2-(4-nitro-benzyl)-piperidin-1-carbonsäurebenzylester
   Analog Beispiel 3b werden 5.0 g (12.6 mMol) N-[1-(4-Nitro-benzyl)-but-3-enyl]-N-ethoxymethyl-carbaminsäure-benzylester mit 1.77 g (25.2 mMol) Chlorsulfonsäure in Acetonitril umgesetzt. Chromatographie an Kieselgel (Methylenchlorid/Methanol (95:5) ergibt die Titelverbindung als farbloses Harz. DC: Methylenchlorid/ Methanol (9:1) R_{f}= 0.40. FD-MS: (M+H)⁺: 412.
d) (2R*,4S*) N-[2-(4-Nitro-benzyl)-piperidin-4-yl]-acetamid
   (2R*,4S*)-4-Acetylamino-2-(4-nitro-benzyl)-piperidin-1-carbonsäurebenzylester (2.65 g, 6.45 mMol) wird mit konz. Salzsäure versetzt und 2 Stunden auf 55°C erhitzt, wobei Gasentwicklung auftritt. Das Reaktionsgemisch wird 2 mal mit Hexan gewaschen und unter vermindertem Druck am Rotationsverdampfer eingedampft. Die Titelverbindung wird als Hydrochlorid in Form weisser Kristalle erhalten, die 3 Moleküle Kristallwasser enthalten.
   Smp.: > 250°C. DC: Methylenchlorid/Methanol/konz. Ammoniak (90:9:1) R_{f}= 0.29. ¹H-NMR (200 MHz, D₂O): δ 8.26 (d, 2H), 7.59 (d, 2H), 4.26-4.17 (m, 1H), 3.,90-3.72 (m, 1H), 3.40-3.02(m, 4H), 1.99 (s, 3H), 2.06-1.75 (m, 4H).
e) (2R*,4S*)-N-[1-(3,5-Bistrifluormethyl-benzoyl)-2-(4-nitro-benzyl)-piperidin-4-yl]-acetamid
   Zu einer gerührten Suspension von 2.50 g (9.0 mMol) (2R*,4S*)-N-[2-(4-Nitro-benzyl)piperidin-4-yl]-acetamid in 20 ml Methylenchlorid und 20 ml einer 10-prozentigen wässrigen Natriumbicarbonatlösung wird bei 0-5°C innerhalb einer Stunde 2.49 g (9,0 mMol) 3,5-Bistrifluormethylbenzoylchlorid gegeben. Anschliessend wird noch 1 Stunde bei 25°C nachgerührt. Die organische Phase wird über Natriumsulfat getrocknet, zur Trockene eingedampft und an Kieselgel mit Methylenchlorid/Methanol chromatographiert. Die Titelverbindung wird als gelbliches Harz erhalten. DC: Methylenchlorid/Methanol/konz. Ammoniak (90:9:0.5) R_{f}= 0.45. ¹H-NMR (200 MHz, D₂O): Rotamerengemisch. δ 8.28-7.08 (m, 7H), 5.41 (br d, NH), 5.36-5.20 (m, 0.5H), 4.87-4.73 (m, 0.5H), 4.51-4.30 (m, 1H), 4.00-3.82 (m, 0.5H), 3.60-2.85 (m, 3.5H), 1.98 (s, 3H), 2.30-1.92 (m, 2H), 1.62-1.22 (m, 2H).
f) (2R*,4S*)-N-Acetyl-N-[1-(3,5-bistrifluormethyl-benzoyl)-2-(4-nitro-benzyl)-piperidin-4-yl]carbaminsäu re-t-butylester
   Zur einer bei 50°C gerührten Lösung von 6.9 g (13.34 mMol) (2R*,4S*)-N-[1-(3,5-Bistrifluormethyl-benzoyl)-2-(4-nitro-benzyl)-piperidin-4-yl]-acetamid, 1.62 g (13.34 mMol) 4-N,N-Dimethylaminopyridin und 2.02 g (20.0 mMol) Triethylamin in 25 ml Toluol werden portionenweise 5.32 g (24.0 mMol) Di-t-butyldicarbonat gegeben. Anschliessend wird noch 18 Stunden bei 50°C gerührt. Nach Abkühlen wird das Reaktionsgemisch mit Ethylacetat verdünnt, mit verdünnter Salzsäure bis pH 3 und danach mit Sole gewaschen. Die organische Phase wird über Natriumsulfat getrocknet, zur Trockne eingedampft und über Kieselgel chromatographiert (Hexan/Ethylacetat 2:1). Die Titelverbindung wird als gelber Feststoff erhalten. DC: Ethylacetat/ Hexan (1:2) R_{f}= 0.40. FD-MS: M⁺: 617.
g) (2R*,4S*)1-(3,5-Bistrifluormethyl-benzoyl)-2-(4-nitro-benzyl)-piperidin-4-amin
   Eine Lösung von 200 mg (0.32 mMol) (2R*,4S*)-N-Acetyl-N-[1-(3,5-Bistrifluormethylbenzoyl)-2-(4-nitro-benzyl)-piperidin-4-yl]-carbaminsäure-t-butylester wird zu einer unter Inertgas gerührten Lösung von 0.04 ml (0.06 mMol) n-Butyllithium in Hexan (1.6 M) in 5 ml Methanol gegeben. Nach 1 Stunde werden 5 mg (0.08 mMol) Essigsäure dazu gegeben und das Gemisch zur Trockne eingedampft. Der Rückstand wird in 2 ml Methylenchlorid und 1.2 ml Trifluoressigsäure aufgenommen und 2.5 Stunden bei Raumtemperatur gerühr Das Gemisch wird mit Methylenchlorid verdünnt und mit 2 N Natronlauge gewaschen. Die organische Phase wird über Natriumsulfat getrocknet, abfiltriert und zur Trockene eingeengt. Die Titelverbindung wird als beiger Schaum erhalten. DC: Dichlormethan/Methanol/konz. Ammoniak (90 : 9 : 0.5) R_{f}= 0.38. ¹H-NMR (200 MHz, D₂O): Rotamerengemisch; δ: 8.28-8.08 (m, 2H), 7.94-7.79 (m, 1H), 7.60-7.40 (m, 2H), 7.21-7.02 (m, 2H), 5.40-5.22 (m, 0.5H), 4.85-4.68 (m, 0.5H), 4.00-3.86 (m, 0.5H), 3.56-3.00 (m, 3H), 2.82-2.70 (m, 0.5H), 2.20-1.86 (m, 2H), 1.6-1.2 (m, 2H).

Beispiel 7: Tabletten, enthaltend je 50 mg (2R,4S)-N-[1-(3,5-Bistrifluormethyl-benzoyl)-2-benzyl-piperidin-4-yl]-chinolin-4-carboxamid oder ein Salz, z.B. das Hydrochlorid, davon können wie folgt hergestellt werden:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 8: Lacktabletten, enthaltend je 100 mg (2R,4S)-N-[1-(3,5-Bistrifluormethyl-benzoyl)-2-benzyl-piperidin-4-yl]-chinolin-4-carboxamid oder ein Salz, z.B. das Hydrochlorid, davon können wie folgt hergestellt werden:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropyl- methylcellulose und des Schellacks in Methylenchlorid lackiert; Endgewicht der Lacktablette: 283 mg.

Beispiel 9: Gelatinesteckkapseln, enthaltend 100 mg Wirkstoff, z.B. (2R,4S)-N-[1-(3,5-Bistrifluormethyl-benzoyl)-2-benzyl-piperidin-4-yl]-chinolin-4-carboxamid oder ein Salz, z.B. das Hydrochlorid, davon, können z.B. folgendermassen hergestellt werden:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 10: In analoger Weise wie in den vorstehenden Beispielen 7 bis 9 beschrieben kann man auch pharmazeutische Präparate, enthaltend eine andere Verbindung der Formel I bzw. lA oder ein Salz davon gemäss einem der vorhergehenden Herstellungsbeispiele herstellen.

## Patentansprüche

1. Verbindung der Formel 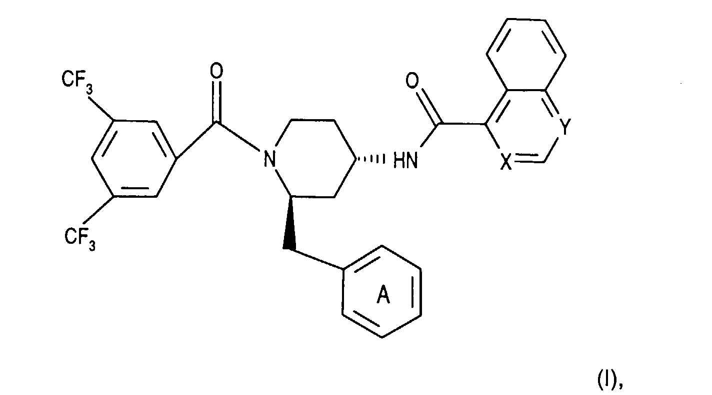 worin X und Y unabhängig voneinander für N oder CH stehen und der Ring A unsubstituiert oder ein- oder mehrfach substituiert ist durch Substituenten ausgewählt aus der Gruppe bestehend aus Niederalkyl, Niederalkoxy, Halogen, Nitro und Trifluormethyl; wobei unter mit "nieder" bezeichneten Resten solche zu verstehen sind, die jeweils 1 bis und mit 7 C-Atome enthalten; oder ein Salz davon.

2. Verbindung gemäss Anspruch 1 der Formel IA 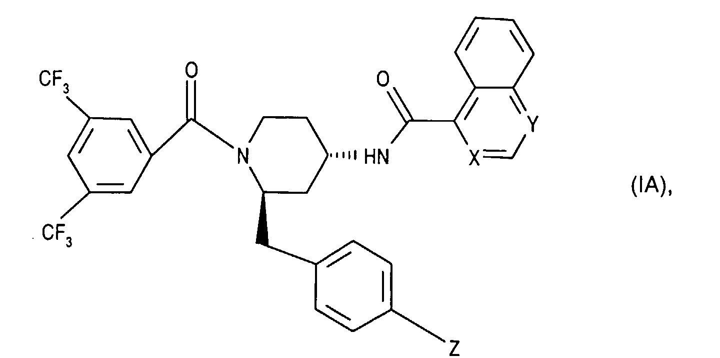 worin X für CH oder N steht und Y N ist; und Z Wasserstoff, Halogen oder Nitro bedeutet; oder ein Salz davon.

3. Verbindung gemäss Anspruch 2 der Formel lA, worin X für N oder CH steht und Y N ist; und Z Halogen bedeutet; oder ein Salz davon.

4. (2R,4S)-N-[1-(3,5-Bistrifluormethyl-benzoyl)-2-(4-chlor-benzyl)-piperidin-4-yl]-chinolin-4-carboxamid; oder ein Salz davon.

5. Eine Verbindung ausgewählt aus der Gruppe bestehend aus
(2R,4S)-N-[1-(3,5-Bistrifluormethyl-benzoyl)-2-benzyl-piperidin-4 chinolin-4-carboxamid;
(2R,4S)-N-[1-(3,5-Bistrifluormethyl-benzoyl)-2-benzyl-piperidin-4-yl]-chinazolin-4-carboxamid;
(2R,4S)-N-[1-(3,5-Bistrifluormethyl-benzoyl)-2-(4-chlor-benzyl)-piperidinyl]-chinazolin-4-carboxamid;
(2R,4S)-N-[1-(3,5-Bistrifluormethyl-benzoyl)-2-(4-chlor-benzyl)-piperidinyl]-isochinolin-1-carboxamid;
(2R,4S)-N-[1-(3,5-Bistrifluormethyl-benzoyl)-2-(4-nitro-benzyl)-piperidinyl]-chinazolin-4-carboxamid;
oder jeweils ein Salz davon.

6. Verbindung gemäss einem der Ansprüche 1-5 oder ein pharmazeutisch verwendbares Salz davon zur Anwendung in einem Verfahren zur Behandlung des menschlichen oder tierischen Körpers.

7. Pharmazeutisches Präparat enthaltend eine Verbindung gemäss einem der Ansprüche 1-5 oder ein pharmazeutisch verwendbares Salz davon.

8. Verwendung einer Verbindung gemäss einem der Ansprüche 1-5 zur Herstellung eines pharmazeutischen Präparates zur Behandlung von Erkrankungen, die durch Substanz P induziert werden.

9. Verwendung gemäss Anspruch 8 zur Herstellung eines pharmazeutischen Präparates zur Behandlung von Zuständen oder Erkrankungen ausgewählt aus Schmerzzuständen, Migräne, Angstzuständen, Emesis, Schizophrenie, Depressionen, morbus Parkinson, rheumatoider Arthritis, Iritis, Konjuntivitis, Asthma, chronischer Bronchitis, ulcerativer Colitis und morbus Crohn.

10. Verfahren zur Herstellung einer Verbindung gemäss einem der Ansprüche 1-5, dadurch gekennzeichnet, dass man
a) eine Verbindung der Formel (IIa) 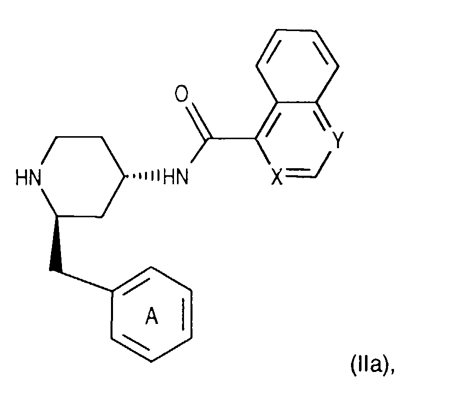 worin X und Y die angegebenen Bedeutungen haben und der Ring A unsubstituiert oder wie angegeben substituiert ist, mit einer Verbindung der Formel llb 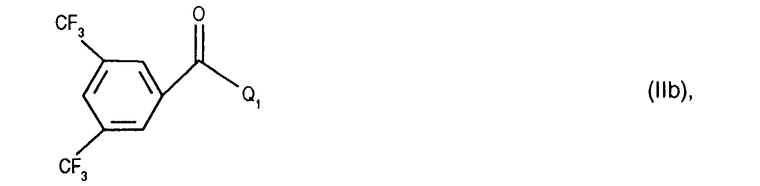 worin Q₁ gegebenenfalls verethertes Hydroxy oder reaktionsfähiges verestertes Hydroxy
oder einen Rest der Formel 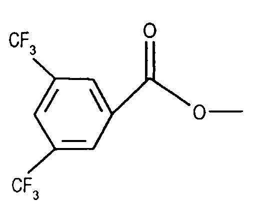 bedeutet, oder einem Salz davon umsetzt; oder
b) eine Verbindung der Formel (IIIa) 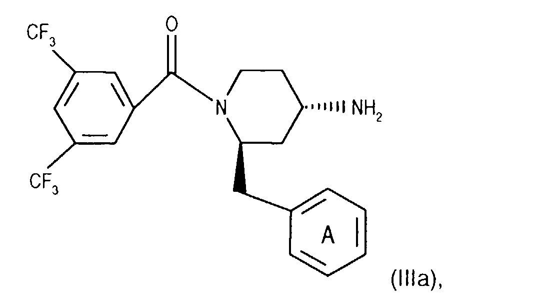 worin der Ring A unsubstituiert oder wie angegeben substituiert ist, mit einer Verbindung der Formel Illb 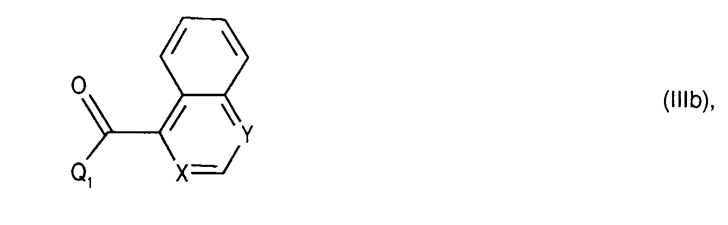 worin X und Y die angegebenen Bedeutungen haben und Q₁ gegebenenfalls verethertes Hydroxy oder reaktionsfähiges verestertes Hydroxy oder einen Rest der Formel 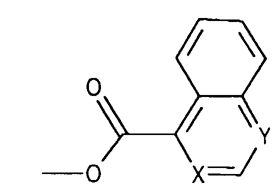 bedeutet, oder einem Salz davon umsetzt;
und, wenn erwünscht, ein verfahrensgemäss erhältliches Gemisch von Isomeren auftrennt und das gewünschte Isomere isoliert und/oder eine verfahrensgemäss erhältliche freie Verbindung I bzw. lA in ein Salz oder ein verfahrensgemäss erhältliches Salz einer Verbindung I bzw. lA in die freie Verbindung I bzw. IA oder in ein anderes Salz überführt.

## Claims

1. A compound of the formula 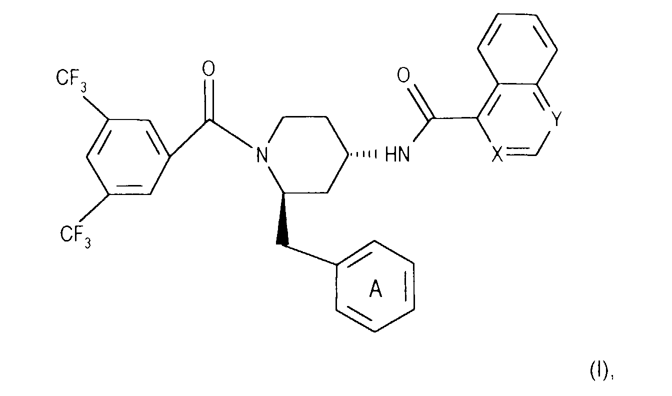 wherein X and Y are each independently of the other N or CH and the ring A is unsubstituted or mono- or poly-substituted by substituents selected from the group consisting of lower alkyl, lower alkoxy, halogen, nitro and trifluoromethyl, wherein radicals referred to as "lower" are to be understood as those each containing from 1 up to and including 7 carbon atoms; or a salt thereof.

2. A compound according to claim 1 of formula IA 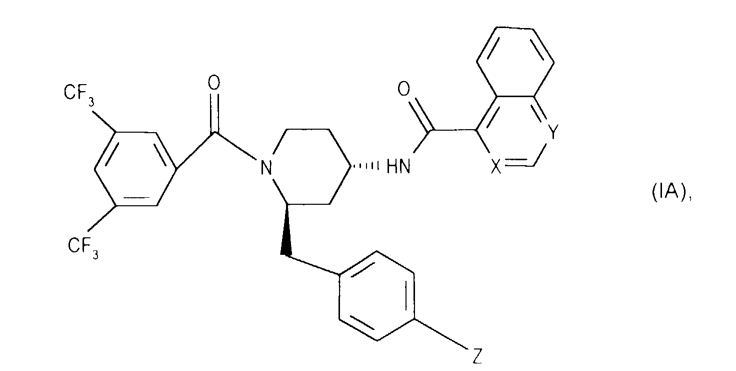 wherein X is CH or N and Y is N, and Z is hydrogen, halogen or nitro, or a salt thereof.

3. A compound according to claim 2 of formula IA, wherein X is N or CH and Y is N, and Z is halogen, or a salt thereof.

4. (2R,4S)-N-[1-(3,5-bis-trifluoromethyl-benzoyl)-2-(4-chloro-benzyl)-piperidin-4-yl]-quinoline-4-carboxamide or a salt thereof.

5. A compound selected from the group consisting of
(2R,4S)-N-[1-(3,5-bis-trifluoromethyl-benzoyl)-2-benzyl-piperidin-4-yl]-quinoline-4-carboxamide;
(2R,4S)-N-[1-(3,5-bis-trifluoromethyl-benzoyl)-2-benzyl-piperidin-4-yl]-quinazoline-4-carboxamide;
(2R,4S)-N-[1-(3,5-bis-trifluoromethyl-benzoyl)-2-(4-chloro-benzyl)-piperidinyl]-quinazoline-4-carboxamide;
(2R,4S)-N-[1-(3,5-bis-trifluoromethyl-benzoyl)-2-(4-chloro-benzyl)-piperidinyl]-isoquinoline-1-carboxamide;
(2R,4S)-N-[1-(3,5-bis-trifluoromethyl-benzoyl)-2-(4-nitro-benzyl)-piperidinyl]-quinazoline-4-carboxamide;
or in each case a salt thereof.

6. A compound according to any one of claims 1 to 5 or a pharmaceutically acceptable salt thereof for use in a method for the treatment of the human or animal body.

7. A pharmaceutical composition comprising a compound according to any one of claims 1 to 5 or a pharmaceutically acceptable salt thereof.

8. The use of a compound according to any one of claims 1 to 5 in the preparation of a pharmaceutical composition for the treatment of disorders induced by substance P.

9. The use according to claim 8 in the preparation of a pharmaceutical composition for the treatment of conditions or disorders selected from painful conditions, migraine, anxiety states, vomiting, schizophrenia, depression, Parkinson's disease, rheumatoid arthritis, iritis, conjunctivitis, asthma, chronic bronchitis, ulcerative colitis and Crohn's disease.

10. A process for the preparation of a compound according to any one of claims 1 to 5, which comprises:
a) reacting a compound of formula (IIa) 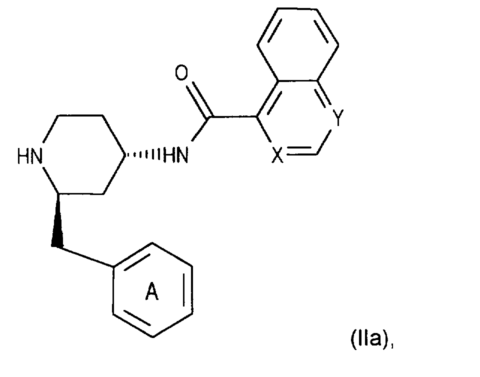 wherein X and Y are as defined and the ring A is unsubstituted or is substituted as indicated, with a compound of formula IIb 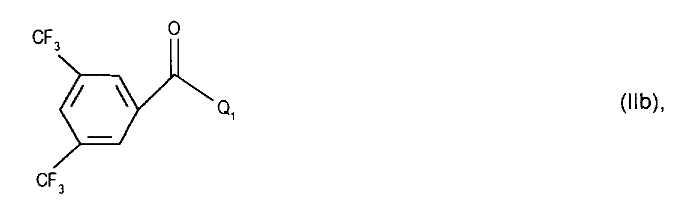 wherein Q₁ is free or etherified hydroxy or reactive esterified hydroxy or a radical of the formula 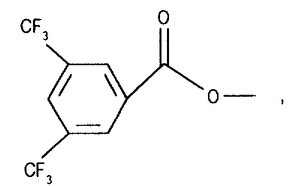 or with a salt thereof; or
b) reacting a compound of formula (IIIa) 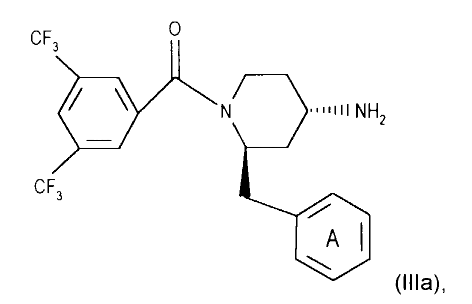 wherein the ring A is unsubstituted or is substituted as indicated, with a compound of formula Illb 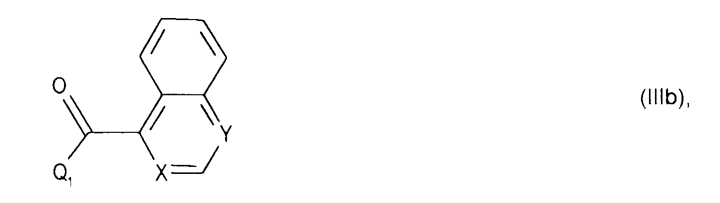 wherein X and Y are as defined and Q₁ is free or etherified hydroxy or reactive esterified hydroxy or a radical of the formula 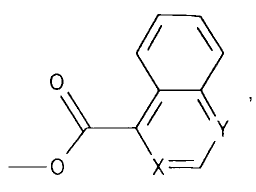 or with a salt thereof;
and, if desired, separating a mixture of isomers obtainable by the process and isolating the desired isomer and/or converting a free compound I or IA obtainable by the process into a salt or converting a salt of a compound I or IA obtainable by the process into the free compound I or IA or into a different salt.

## Revendications

1. Un composé de formule 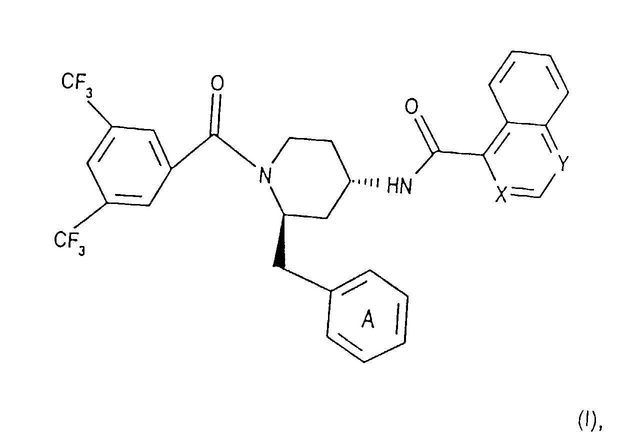 où X et Y signifient indépendamment l'un de l'autre N ou CH et le cycle A est non substitué ou mono- ou polysubstitué par des substituants choisis parmi le groupe comprenant un groupe alkyle inférieur, alcoxy inférieur, un halogène, un groupe nitro et trifluorométhyle, l'expression "inférieur", désignant les restes qui contiennent de 1 à 7 atomes de carbone; ou bien un de ses sels.

2. Un composé selon la revendication 1 de formule IA 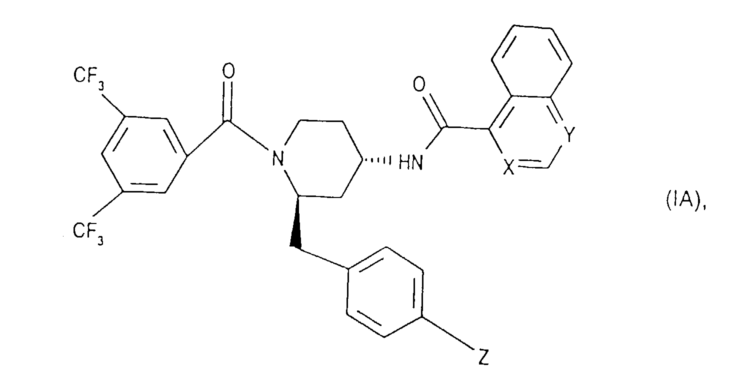 où X signifie CH ou N et Y signifie N; et Z signifie l'hydrogène, un halogène ou un groupe nitro; ou un de ses sels.

3. Un composé selon la revendication 2 de formule IA, où X signifie N ou CH et Y signifie N; et Z signifie un halogène; ou un de ses sels.

4. Le (2R,4S)-N-[1-(3,5-bistrifluorométhyl-benzoyl)-2-(4-chloro-benzyl)pipéridine-4-yl]-quinoline-4-carboxamide; ou un de ses sels.

5. Un composé choisi parmi le groupe comprenant
le (2R,4S)-N-[1-(3,5-bistrifluorométhyl-benzoyl)-2-benzyl-pipéridine-4-yl-quinoline-4-carboxamide;
le (2R,4S)-N-[1-(3,5-bistrifluorométhyl-benzoyl)-2-benzyl-pipéridine-4-yl] -quinazoline-4-carboxamide;
le (2R,4S)-N-[1-(3,5-bistrifluorométhyl-benzoyl)-2-(4-chloro-benzyl)-pipéridinyl]quinazoline-4-carboxamide;
le (2R,4S)-N-[1-(3 5-bistrifluorométhyl-benzoyl)-2-(4-chloro-benzyl)-pipéndinyl]-isoquinoline-1-carboxamide;
le (2R,4S)-N-[1-(3,5-bistrifluorométhyl-benzoyl)-2-(4-nitro-benzyl)-pipéridinyl]quinazoline-4-carboxamide;
ou un de ses sels.

6. Un composé selon l'une quelconque des revendications 1 à 5 ou un de ses sels pharmaceutiquement acceptables pour une utilisation dans une méthode de traitement du corps humain ou animal.

7. Une composition pharmaceutique contenant un composé selon l'une quelconque des revendications 1 à 5 ou un de ses sels pharmaceutiquement acceptables.

8. Utilisation d'un composé selon l'une quelconque des revendications 1 à 5 pour la préparation d'une composition pharmaceutique pour le traitement de troubles induits par la substance P.

9. Utilisation selon la revendication 8, pour la préparation d'une composition pharmaceutique pour le traitement de conditions ou de maladies choisies parmi les états douloureux, la migraine, les états d'anxiété, les vomissements, la schizophrénie, la dépression, la maladie de Parkinson, l'arthrite rhumatoïde, l'iritis, la conjonctivite, l'asthme, la bronchite chronique, la colite ulcérative et la maladie de Crohn.

10. Un procédé de préparation d'un composé selon l'une quelconque des revendications 1 à 5, caractérisé en ce qu'on fait réagir
a) un composé de formule (IIa) 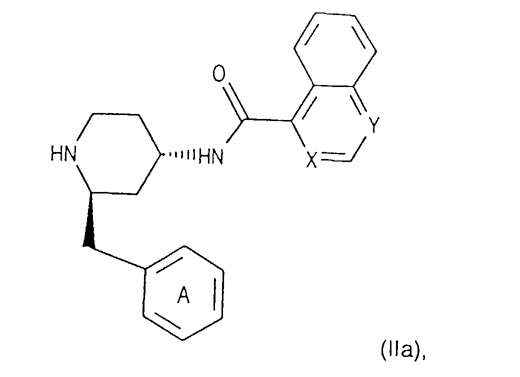 où X et Y ont les significations données et le cycle A est non substitué ou substitué comme indiqué, avec un composé de formule IIb 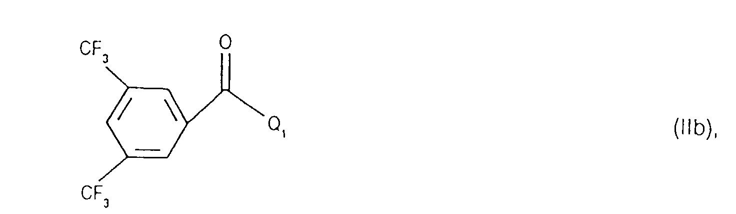 où Q₁ signifie un groupe hydroxy éventuellement éthérifié ou un groupe hydroxy estérifié réactif
ou bien un reste de formule 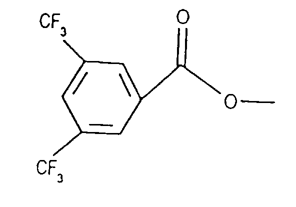 ou bien un de ses sels; ou bien
b) on fait réagir un composé de formule (IIIa) 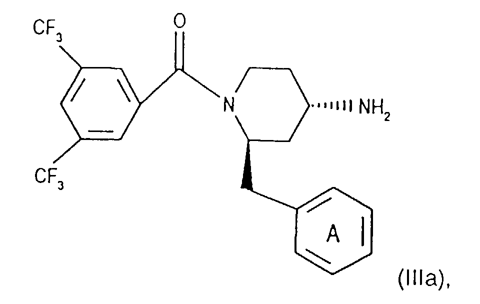 où le cycle A est non substitué ou substitué comme indiqué, avec un composé de formule IIIb 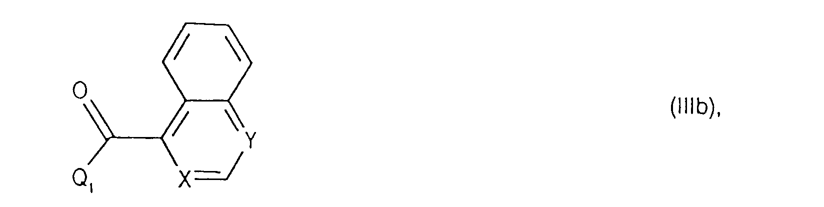 où X et Y ont les significations indiquées et Q₁ signifie un groupe hydroxy éventuellement éthérifié ou un groupe hydroxy estérifié réactif ou bien un reste de formule 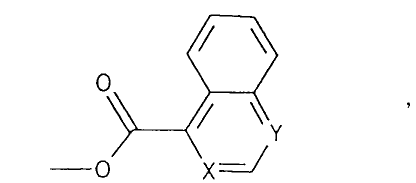 ou bien un de ses sels;
et, lorsque cela est nécessaire, on sépare un mélange d'isomères obtenu selon le procédé et on isole l'isomère désiré et/ou on transforme un composé libre I ou IA obtenu selon le procédé en un sel ou bien on transforme un sel d'un composé I ou IA obtenu selon le procédé en un composé libre I ou IA ou en un autre sel.
